# EUROPEAN PATENT APPLICATION

(11) **EP 4 190 797 A1**
(43) Date of publication of application: **07.06.2023**
(21) Application number: 21850367.0
(22) Date of filing: 07.07.2021
(51) Int. Cl.: C07K 7/06, A61K 38/08, A61K 39/395, A61P 25/00, A61P 25/16, A61P 25/28, A61P 43/00, C07K 16/18, C12N 15/12

(54) **PEPTIDE USED TO PREVENT OR TREAT SYNUCLEINOPATHY**

(30) Priority: 29.07.2020 JP 2020127958
(71) Applicant: Tohoku University, Sendai-shi, Miyagi 980-8577 (JP)
(72) Inventor: FUKUNAGA, Koji, Sendai-shi, Miyagi 980-8577 (JP); KAWAHATA, Ichiro, Sendai-shi, Miyagi 980-8577 (JP); KAWATA, Yasushi, Tottori-shi, Tottori 680-8550 (JP); MIZOBATA, Tomohiro, Tottori-shi, Tottori 680-8550 (JP); FUKUI, Naoya, Tottori-shi, Tottori 680-8550 (JP)
(74) Representative: Winter, Brandl - Partnerschaft mbB
(86) International application number: PCT/JP2021/025577
(87) International publication number: WO 2022/024693

(57) **Abstract**

A peptide represented by (i) or (ii) below:
(i) a peptide having an amino acid sequence represented by EEG(X1)QD(X2)EPEA, wherein X1 and X2 are identical or different and are Y, F, or W; or
(ii) a peptide having an amino acid sequence where one or several amino acids are deleted, substituted, or added in amino acid positions excluding X1 and X2 of the amino acid sequence of (i) above and having an activity of binding to FABP3 protein.

## Description

### TECHNICAL FIELD

The present invention relates to a peptide for use in prevention or treatment of synucleinopathy.

### BACKGROUND ART

α-Synuclein is said to be a causal protein for synucleinopathies (Parkinson's disease, dementia with Lewy bodies, and multiple system atrophy), and synucleinopathies are characterized by aggregation of α-synuclein in nerve cells and glial cells. In a neuropathological study of synucleinopathy, characteristic damage including abnormal aggregation of α-synuclein can be detected in nerve cells, nerve fibers, or glial cells, α-synuclein propagates between nerve cells or between glial cells and aggregates/accumulates, and this is known to cause necrosis of nerve cells or glial cells throughout the brain and to lead to motor impairment, cognitive impairment, sleep disturbance, and/or autonomic neuropathy. Antibody therapy for α-synuclein is currently being developed but has not been established yet as a fundamental treatment because of side-effect problems.

Patent Literature 1 discloses an antibody specifically binding to human α-synuclein and a pharmaceutical formulation containing such an antibody and inhibiting cytotoxicity.

Patent Literature 2 discloses a pharmaceutical composition for prevention and/or treatment of synucleinopathy, the composition containing a peptide composed of nine amino acids, the peptide containing an amino acid sequence considered to be a mimotope of an epitope of α-synuclein.

Non-Patent Literature 1 discloses that FABP3 enhances diffusion of α-synuclein after injection of pre-formed fibrils of α-synuclein in the mouse brain and mediates α-synuclein toxicity in synucleinopathy conditions, and MF1, an FABP3 ligand, is an attractive treatment candidate for α-synucleinopathy.

Non-Patent Literature 2 is a review on cleavage and disease of α-synuclein and defines the N-terminal region (residues 1-60), NAC region (residues 61-95), and C-terminal region (residues 96-140) of α-synuclein. This describes that cleavage at the C-terminal side of α-synuclein increases the aggregation rate of the cleaved α-synuclein.

Non-Patent Literature 3 discloses development of amido-bridged nucleic acids-modified antisense oligonucleotide (AmNA-ASO) that inhibits accumulation of α-synuclein protein and improvement of symptoms of Parkinson's disease in an animal model.

### CITATION LIST

### Patent Literature

Patent Literature 1: JP 2020-73565 A
Patent Literature 2: JP 5901714 B

### Non-Patent Literature

Non-Patent Literature 1: Int. J. Mol. Sci. 2020 Mar 23; 21(6): 2230
Non-Patent Literature 2: JBC Papers in Press, on May 18, 2020 as Manuscript REV120.011743
Non-Patent Literature 3: Scientific Reports (2019) 9: 7567

### SUMMARY OF INVENTION

### Technical Problem

Objects to be solved by the present invention are to provide a peptide and an antibody for use in prevention or treatment of synucleinopathy; a preventive or therapeutic agent for prevention or treatment of synucleinopathy, the agent containing a peptide or an antibody; and a pharmaceutical composition for prevention or treatment of synucleinopathy, the composition containing a peptide or an antibody.

### Solution to Problem

As a result of diligent studies to solve the above objects, the present inventors focused on complex formation by α-synuclein and FABP3, found that using a peptide derived from the C-terminal region of α-synuclein can inhibit uptake of α-synuclein into nerve and glial cells and/or propagation of α-synuclein between nerve and glial cells, and completed the present invention.

The present invention includes embodiments described below.

Item 1. A peptide represented by (i) or (ii) below:
(i) a peptide having an amino acid sequence represented by EEG(X1)QD(X2)EPEA, wherein X1 and X2 are identical or different and are Y, F, or W; or
(ii) a peptide having an amino acid sequence where one or several amino acids are deleted, substituted, or added in amino acid positions excluding X1 and X2 of the amino acid sequence of (i) above and having an activity of binding to FABP3 protein.

Item 2. The peptide according to item 1, to bind to FABP3 through an interaction between phenyl groups of the amino acids of X1 and X2 and a phenyl group of 16th phenylalanine in an amino acid sequence of native FABP3 protein.

Item 3. An antibody against the peptide according to claim 1 or 2.

Item 4. A preventive or therapeutic agent for prevention or treatment of synucleinopathy, the agent containing the peptide according to claim 1 or 2 or the antibody according to claim 3 as an active ingredient.

Item 5. The preventive or therapeutic agent according to claim 4, wherein the synucleinopathy is selected from the group consisting of Parkinson's disease, Lewy body disease, and multiple system atrophy.

Item 6. A pharmaceutical composition for prevention or treatment of synucleinopathy, the composition containing the peptide according to claim 1 or 2 or the antibody according to claim 3.

### BRIEF DESCRIPTION OF DRAWINGS

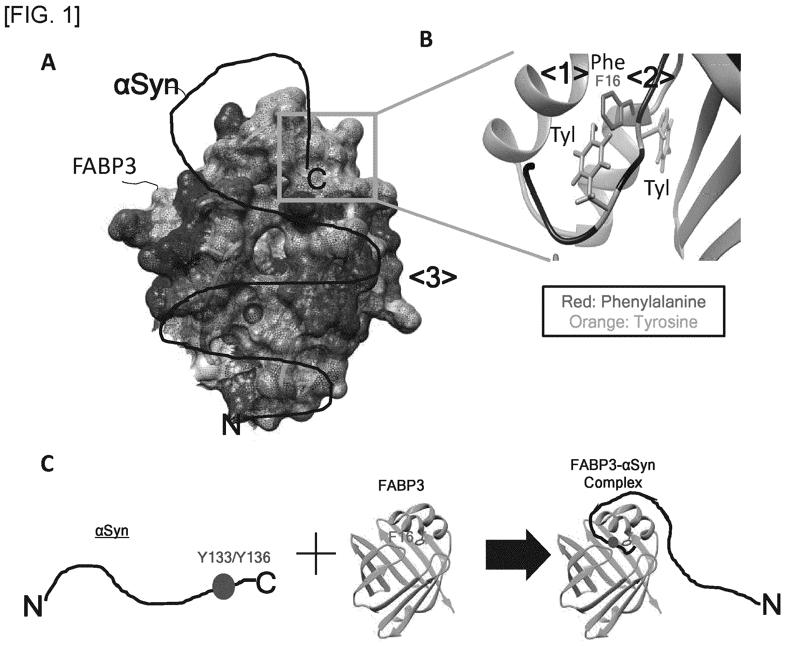

FIGS. 1A to 1C are schematic or enlarged views illustrating a three-dimensional structure and a bonding site of α-synuclein and FABP3. FIG. 1A is a schematic view to explain an interaction between α-synuclein and FABP3. FIG. 1B is an enlarged view of the part surrounded by the square in FIG. 1A of the C-terminus of α-synuclein. FIG. 1C is a schematic view of a binding mode of α-synuclein with a denatured structure and FABP3. The 133rd and 136th tyrosine residues of α-synuclein are involved in the binding mode. Especially the 133rd tyrosine residue strongly interacts with a phenyl group of the 16th phenylalanine located in a fatty acid-binding pocket of FABP3, forming a complex of α-synuclein and FABP3.

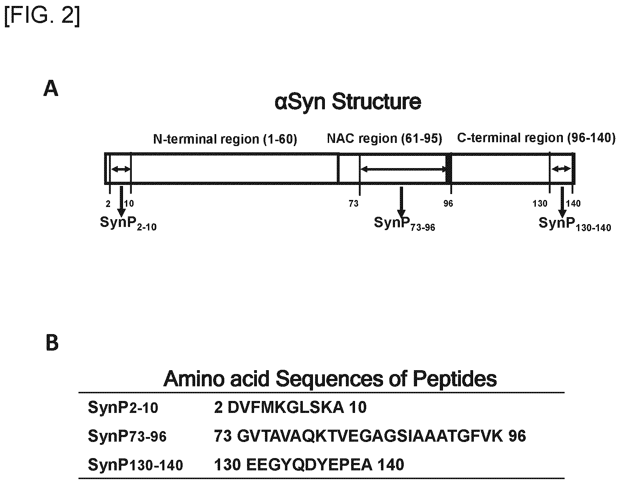

FIGS. 2A and 2B are a schematic view illustrating a two-dimensional structure and a list of characteristics of each domain of α-synuclein. FIG. 2A is a schematic view illustrating structures of the N-terminus, NAC-terminus, and C-terminus of α-synuclein. FIG. 2B is a list of amino acid sequences of peptides each in each region in FIG. 2A.

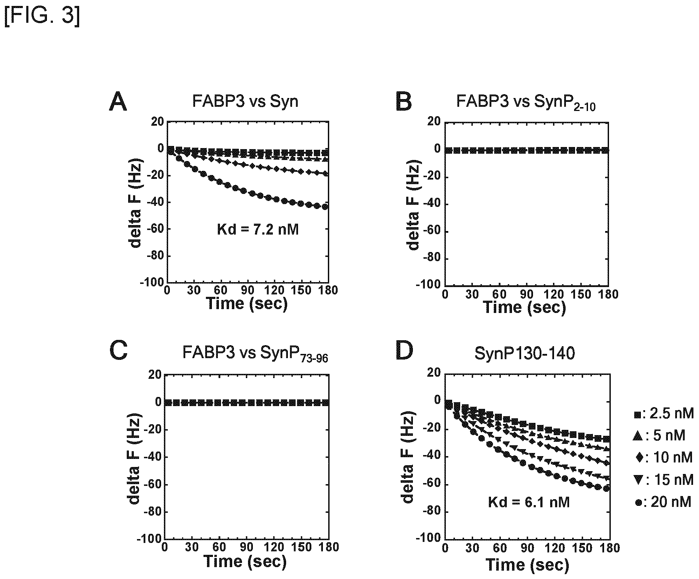

FIGS. 3A to 3D are graphs showing results of binding analysis of FABP3 and α-synuclein or its domain. FIG. 3A is a graph showing results of binding analysis of FABP3 and full length α-synuclein over time. FIG. 3B is a graph showing results of binding analysis of FABP3 and SynP₂₋₁₀ over time. FIG. 3C is a graph showing results of binding analysis of FABP3 and SynP₇₃₋₉₆ over time. FIG. 3D is a graph showing results of binding analysis of FABP3 and SynP₁₃₀₋₁₄₀ over time.

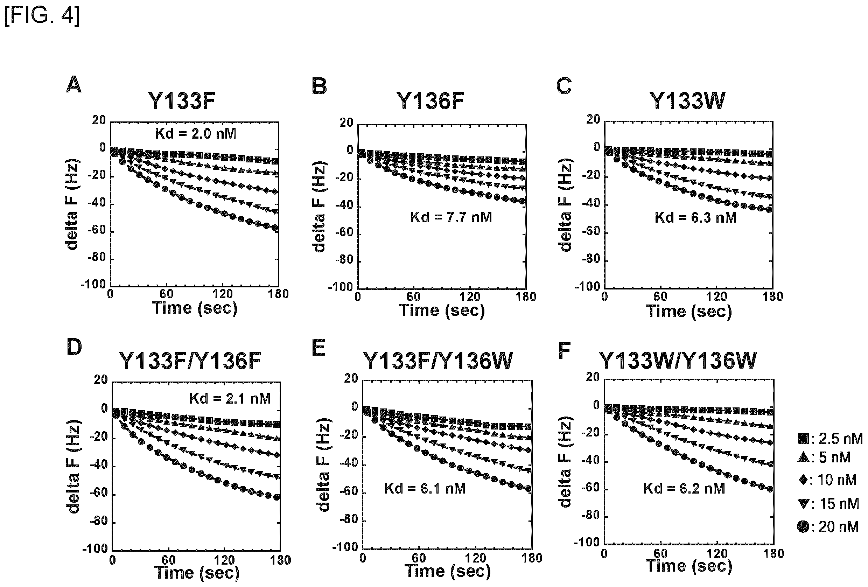

FIGS. 4A to 4F are graphs showing results of binding analysis of ABP3 and various mutant α-synucleins. FIG. 4A is a graph showing results of binding analysis of FABP3 and a peptide in which the 133rd tyrosine in SynP₁₃₀₋₁₄₀ was replaced by phenylalanine (Y133F). FIG. 4B is a graph showing results of binding analysis of FABP3 and a peptide in which the 136th tyrosine in SynP₁₃₀₋₁₄₀ was replaced by phenylalanine (Y136F). FIG. 4C is a graph showing results of binding analysis of FABP3 and a peptide in which the 136th tyrosine in SynP₁₃₀₋₁₄₀ was replaced by tryptophan (Y136W). FIG. 4D is a graph showing results of binding analysis of FABP3 and a peptide in which the 133rd and 136th tyrosines in SynP₁₃₀₋₁₄₀ were replaced by phenylalanines (Y133F/Y136F). FIG. 4E is a graph showing results of binding analysis of FABP3 and a peptide in which the 133rd tyrosine in SynP₁₃₀₋₁₄₀ was replaced by phenylalanine and the 136th tyrosine in SynP₁₃₀₋₁₄₀ was replaced by tryptophan (Y133F/Y136W). FIG. 4F is a graph showing results of binding analysis of FABP3 and a peptide in which the 133rd and 136th tyrosines in SynP₁₃₀₋₁₄₀ were replaced by tryptophans (Y133W/Y136W).

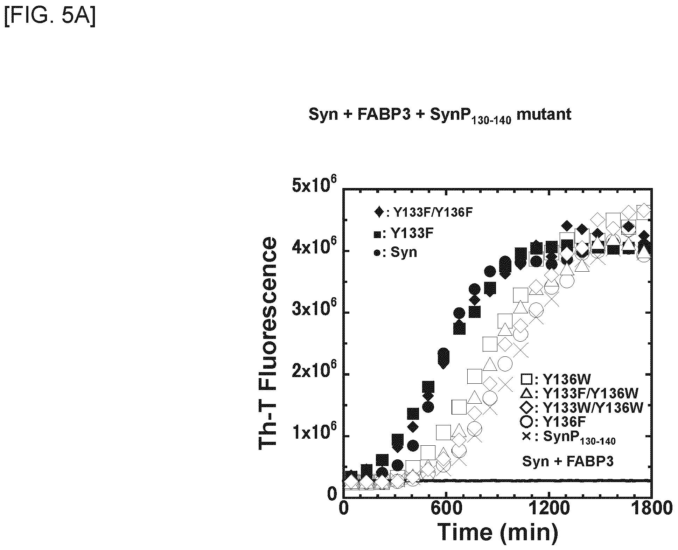

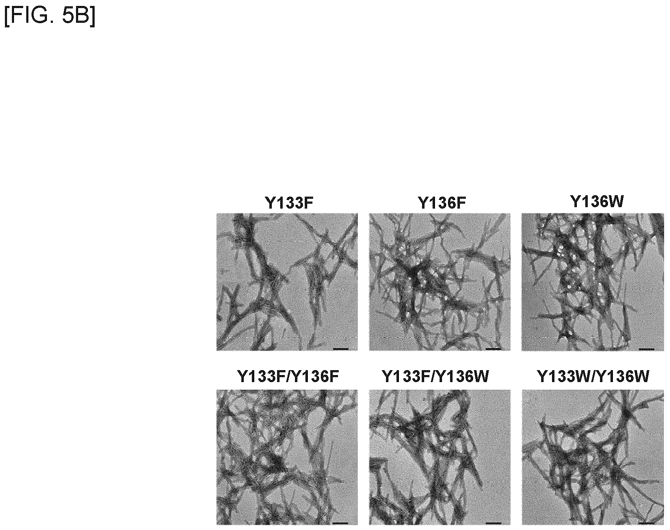

FIGS. 5A and 5B are a graph showing results of binding analysis of FABP3 and each C-terminal deletion/mutant α-synuclein and electron microscopic images. FIG. 5A is a graph showing synuclein aggregate formation when equimolar FABP3 and SynP₁₃₀₋₁₄₀ or its mutant peptide were added simultaneously to α-synuclein, where each symbol indicates results of adding each of the following peptides: ◆ Y133F/Y136F, ■ Y133F, ● Syn full length, □ Y136W, △ Y133F/Y136W, ◊ Y133W/Y136W, ∘ Y136F, and × SynP₁₃₀₋₁₄₀. FIG. 5B is a series of micrographs showing synuclein aggregation when each SynP₁₃₀₋₁₄₀ mutant peptide was added.

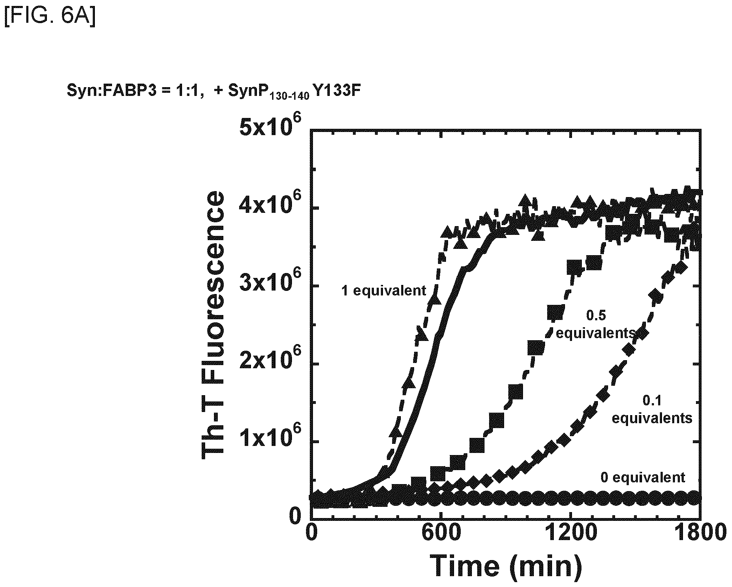

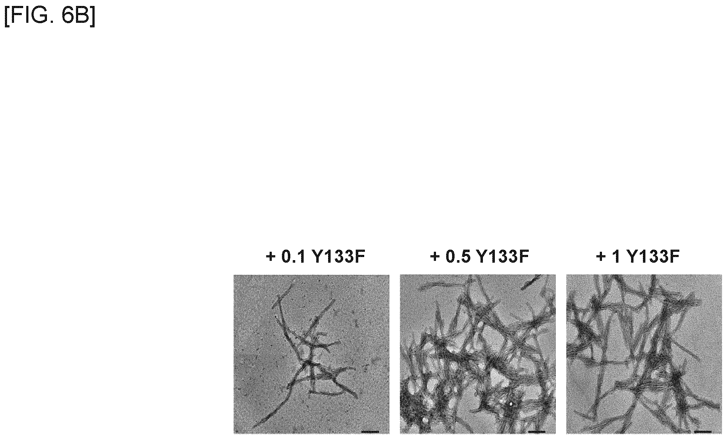

FIGS. 6A and 6B are a graph showing antagonistic effects by α-synuclein C-terminal peptides on binding of synuclein and FABP3 and electron microscopic images. FIG. 6A is a graph showing synuclein aggregate formation when equimolar FABP3 and 1 equivalent, 0.5 equivalents, or 0.1 equivalents of a mutant peptide Y133F of SynP₁₃₀₋₁₄₀ were added simultaneously to α-synuclein. FIG. 6B is a series of micrographs showing synuclein aggregation at each Y133F concentration (▲ 1 equivalent, ■ 0.5 equivalents, ◆ 0.1 equivalents, and • 0 equivalent).

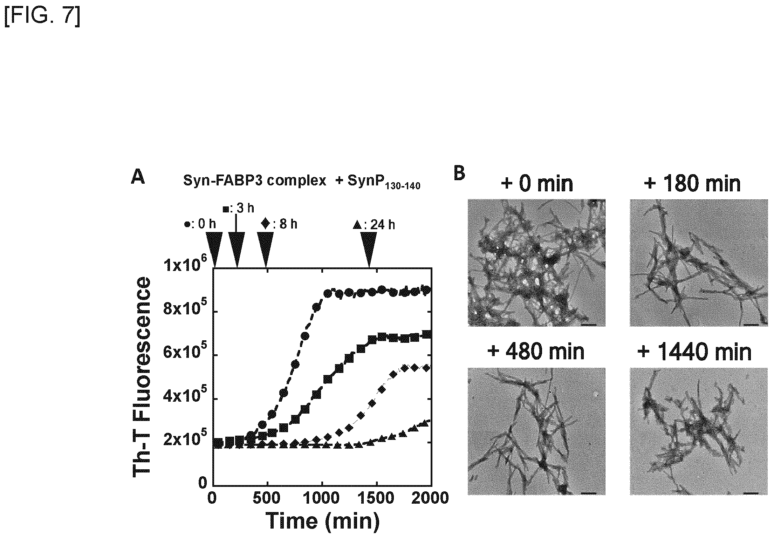

FIGS. 7A and 7B are a graph showing time-dependent antagonistic effects by α-synuclein C-terminal peptides on synuclein and FABP3 and electron microscopic images. FIG. 7A is a graph showing synuclein aggregate formation when equimolar FABP3 was added to α-synuclein to form a Syn-FABP3 complex and then a SynP₁₃₀₋₁₄₀ peptide was added. FIG. 7B is a series of micrographs showing synuclein aggregation after 0 minutes (●), 180 minutes (■), 480 minutes (◆), and 1440 minutes (▲) of the addition of FABP3, each time point indicated by the reverse triangle (▼) in FIG. 7A.

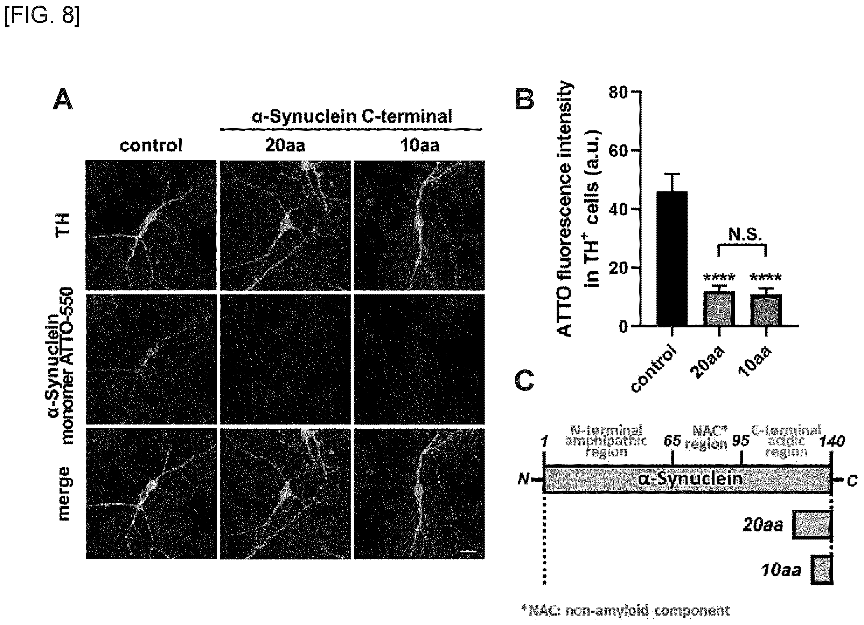

FIGS. 8A to 8C are images and graphs showing uptake inhibition of an α-synuclein monomer into FABP-expressing nerve cells by C-terminal region peptides of α-synuclein, and a schematic view indicating the locations of the C-terminal region peptides. FIG. 8A is a series of images showing results of controls (addition of α-synuclein) in the left row, results of adding a peptide (20aa) composed of 20 amino acids of the C-terminal region of α-synuclein in the middle row, and results of adding a peptide (10aa) composed of 10 amino acids of the C-terminal region of α-synuclein in the right row, where images for tyrosine hydroxylase (green), a rate determining enzyme for dopamine, are shown in the upper line, images of fluorescence labeling of an α-synuclein monomer with ATTO-550 (red) are shown in the middle line, and merges of the images in the upper and middle lines are shown in the bottom line. FIG. 8B is a graph showing ATTO fluorescence intensity of each of the control, the 20aa peptide addition, and the 10aa peptide addition, where **** indicates p < 0.0001. FIG. C is a schematic view indicating the locations of the 20aa peptide and 10aa peptide in the α-synuclein C-terminal region.

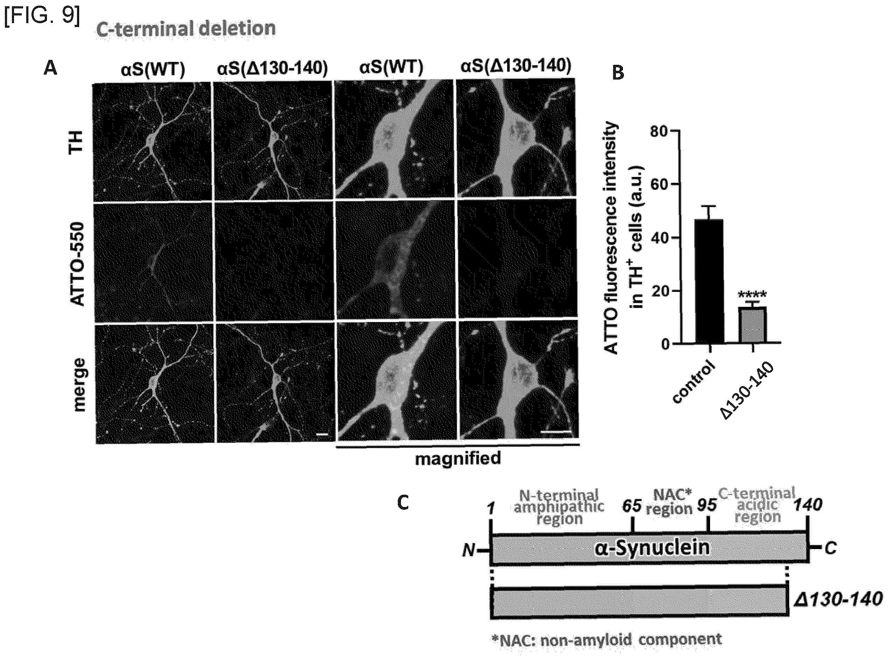

FIGS. 9A to 9C are images and graphs showing uptake inhibition of an α-synuclein monomer into FABP-expressing nerve cells by C-terminal deletion peptides of α-synuclein, and a schematic view illustrating the C-terminal deletion peptides. FIG. 9A is a series of images for a wild-type α-synuclein monomer (αS (WT) ) and a peptide in which the 130th to 140th amino acids of the wild-type α-synuclein sequence were deleted (αS(Δ130-140)), where images for tyrosine hydroxylase (green) are shown in the upper line, images of fluorescence labeling with ATTO-550 are shown in the middle line, and merges of images in the upper and middle lines are shown in the bottom line. FIG. 9B is a graph showing ATTO fluorescence intensity of each of the control (αS(WT)) and αS(Δ130-140), where **** indicates p < 0.0001. FIG. 9C is a schematic view illustrating a sequence of the αS(Δ130-140) peptide.

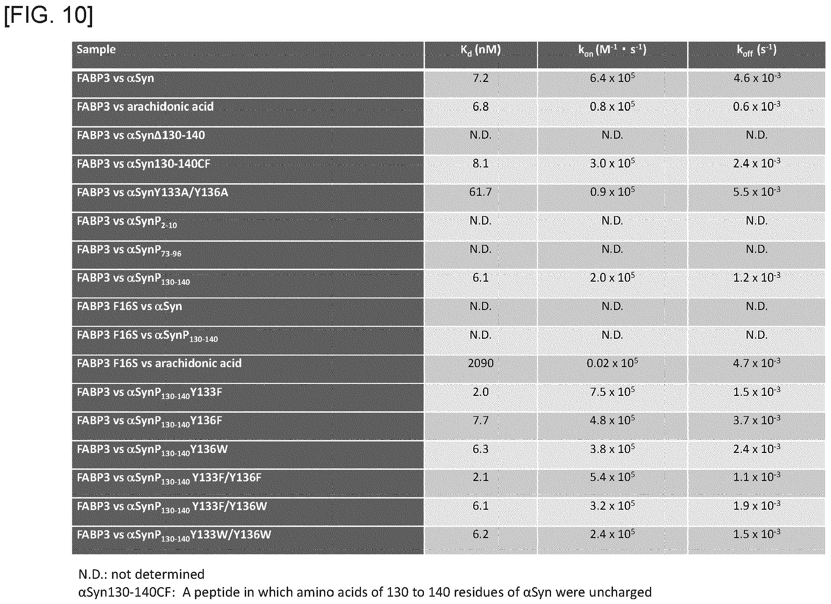

FIG. 10 is a table showing a list of FABP3 and various mutant/deletion α-synuclein or peptides and their dissociation constants, and Kd, kₒₙ, and k_{off} values in a binding experiment of FABP3 protein and various α-synuclein peptides. AA: arachidonic acid. FABP3 F16S: a protein in which the 16th phenyl alanine of wild-type FABP3 was replaced by serine.

### DESCRIPTION OF EMBODIMENTS

α-Synuclein is fibrillized and aggregated from a monomer to an oligomer then to a multimer, such as those of protofibrils, in the brain. According to the recent study, oligomerization of α-synuclein is believed to be involved in the propagation between nerve cells or between glial cells and to exhibit cytotoxicity. The inventor of the present application recently reported that when α-synuclein forms an oligomer, fatty acid-binding protein 3 (FABP3) also produces an oligomer together with α-synuclein (Kawahata, I. et al., Int. J. Mol. Sci. 2019, 20, (21)). As the disease progresses, oligomerized α-synuclein propagates between nerve cells, where it is taken up by nerve cells and causes cell death. FABP3 is involved in this uptake of α-synuclein into nerve and glial cells. That is, oligomerization by FABP3 and α-synuclein appears to be involved in both propagation and uptake of α-synuclein, and inhibition or suppression of the interaction between FABP3 and α-synuclein if feasible would be useful in the prevention or treatment of synucleinopathy.

Thus, the inventors of the present application produced peptides each corresponding to the N-terminal region (region composed of the second to tenth amino acids from the N-terminus, SEQ ID NO: 2), the NAC region (region composed of the 73th to 96th amino acids, SEQ ID NO: 3), or the C-terminal region (region composed of the 130th to 140th amino acids, SEQ ID NO: 4) of human α-synuclein protein (140 amino acids, SEQ ID NO: 1, UniPlot: P37840) and examined which region of α-synuclein is involved in the interaction with FABP3.

As a result, the inventors of the present application surprisingly found that neither the N-terminal region nor the NAC region but the C-terminal region is largely involved in the interaction with FABP3.

FIGS. 1A to C are schematic views of formation of a 1:1 complex of α-synuclein and FABP3. The C-terminal region of α-synuclein indicated by one curve binds to the fatty acid-binding site of FABP3. The 16th Phe of FABP3 and the 133rd Tyr in the C-terminal region of α-synuclein are largely involved in the binding. In addition, changing the 133rd Tyr of the peptide of α-synuclein to Phe provides stronger binding. Regions of α-synuclein other than the C-terminal presumably binds to the surface of FABP3 through electric charge or the like.

According to a first aspect of the present invention, there is provided a peptide represented by (i) or (ii) below:
(i) a peptide having an amino acid sequence represented by EEG(X1)QD(X2)EPEA (SEQ ID NO: 5),
   where X1 and X2 are identical or different and tyrosine (Y), phenylalanine (F), or tryptophan (W); or
(ii) a peptide having an amino acid sequence where one or several amino acids are deleted, substituted, or added in amino acid positions excluding X1 and X2 of the amino acid sequence of (i) above and having an activity of binding to FABP3 protein.

Amino acid symbols in the present specification are according to IUPAC amino acid abbreviations.

One-letter codes, three-letter codes, and amino acids are shown below:

| | | | |
|---|---|---|---|
| A, Ala: | alanine | M, Met: | methionine |
| C, Cys: | cysteine | N, Asn: | asparagine |
| D, Asp: | aspartic acid | P, Pro: | proline |
| E, Glu: | glutamic acid | Q, Gln: | glutamine |
| F, Phe: | phenylalanine | R, Arg: | arginine |
| G, Gly: | glycine | S, Ser: | serine |
| H, His: | histidine | T, Thr: | threonine |
| I, Ile: | isoleucine | V, Val: | valine |
| K, Lys: | lysine | W, Trp: | tryptophan |
| L, Leu: | leucine | Y, Tyr: | tyrosine |

The peptide of the first aspect of the present invention includes not only the peptide represented by (i) or (ii) where each amino acid is unmodified but also a peptide represented by (i) or (ii) where one or a plurality of amino acids is modified while the type of amino acids remains maintained. Such modification includes oxidation by binding of an oxygen atom, phosphorylation, N-acetylation, and S-cysteination.

The peptide of the present invention binds to FABP3 through an interaction between phenyl groups of the amino acids of X1 and X2 and a phenyl group of 16th phenylalanine in an amino acid sequence of native FABP3 protein.

In one embodiment, the peptide of the present invention is a peptide represented by (i) above, where X1 is tyrosine, and X2 is tyrosine, phenylalanine, or tryptophan.

In another embodiment, the peptide of the present invention is a peptide represented by (i) above, where X1 is phenylalanine, and X2 is tyrosine, phenylalanine, or tryptophan. The peptide where X1 is phenylalanine has an increased binding affinity for FABP3 protein compared with the peptide where X1 is tyrosine.

In another embodiment, the peptide of the present invention is a peptide represented by (i) above, where X1 is tryptophan, and X2 is tyrosine, phenylalanine, or tryptophan.

In one embodiment, the peptide of the present invention is a peptide represented by (ii) above having an amino acid sequence where one or two amino acids are deleted, substituted, or added in amino acid positions and having an activity of binding to FABP3 protein.

In the peptide where one or two amino acids are substituted or added in the amino acid positions, such substituted or added amino acids may be natural amino acids or may be unnatural amino acids (i.e., amino acids other than the 20 classical amino acids).

In another embodiment, the peptide of the present invention is a peptide represented by (ii) above having an amino acid sequence where one amino acid is deleted, substituted, or added in amino acid positions and having an activity of binding to FABP3 protein.

In another embodiment, the peptide of the present invention is a peptide represented by (ii) above having an amino acid sequence where one amino acid is deleted, substituted, or added in amino acid positions and having an activity of binding to FABP3 protein; and X1 is tyrosine and X2 is tyrosine, phenylalanine, or tryptophan; X1 is phenylalanine and X2 is tyrosine, phenylalanine, or tryptophan; or X1 is tryptophan and X2 is tyrosine, phenylalanine, or tryptophan.

In one embodiment, the peptide of the present invention is a peptide represented by (i) or (ii) above, where the amino acid length is from 7 to 30, preferably from 8 to 20, more preferably from 9 to 20, even more preferably from 10 to 20, and for example, 9, 10, 11, 12, 13, 14, 15, or 16.

The peptide of the first aspect of the present invention can bind to FABP3, thus competes with α-synuclein, and can inhibit or suppress the interaction between FABP3 and α-synuclein. The inhibitory or suppressive effect of the peptide of the first aspect of the present invention on the interaction between α-synuclein and FABP3 can be determined by in vitro inhibition assay. When the interaction between α-synuclein and FABP3 is inhibited or suppressed, the oligomerization by α-synuclein and FABP3 is inhibited, and thus this suppresses the propagation of α-synuclein between nerve cells and the uptake of α-synuclein into FABP3-expressing nerve cells. Thus, the peptide of the first aspect of the present invention appears to be useful for the prevention or treatment of synucleinopathy.

The peptide of the first aspect of the present invention can be manufactured as a peptide isolated by a chemical synthesis method known to those skilled in the art or can also be manufactured as a part of another peptide.

According to a second aspect of the present invention, there is provided an antibody against the peptide of the first aspect of the present invention. Such an antibody is specific for the peptide of the first aspect and recognizes a part or whole of the peptide of the first aspect of the present invention as an epitope, and thus inhibits or suppresses the interaction between α-synuclein and FABP3. Thus, the antibody of the second aspect of the present invention also appears to be useful for the prevention or treatment of synucleinopathy.

The antibody of the second aspect of the present invention can be produced by manufacturing the peptide of the first aspect of the present invention by a chemical synthesis method known to those skilled in the art and subjecting the peptide as an antigen to a known immunological technique. The antibody of the second aspect of the present invention may be either a polyclonal antibody or a monoclonal antibody. In addition, the antibody includes not only a complete antibody molecule but also its fragment and may be, for example, Fab, F(ab')2, or ScFv.

The peptide of the first aspect of the present invention is derived from a partial sequence of α-synuclein, and thus the peptide of the first aspect and the antibody of the second aspect of the present invention can inhibit the uptake of α-synuclein into FABP3-expressing nerve cells but can avoid side effects. In addition, a peptide and an antibody that are stable in the living body and easily transferred into the brain can be designed.

According to a third aspect of the present invention, there is provided a method of inhibiting the interaction between α-synuclein and FABP3, the method including exposing α-synuclein to the peptide of the first aspect or the antibody of the second aspect of the present invention.

The inhibition method may be a method in vivo or may be a method in vitro.

According to a fourth aspect of the present invention, there is provided a method of inhibiting the uptake of α-synuclein into FABP3-expressing cells, the method including exposing α-synuclein to the peptide of the first aspect or the antibody of the second aspect of the present invention.

The inhibition method may be a method in vivo or may be a method in vitro. Examples of the FABP3-expressing cell include, but are not limited to, dopaminergic nerve cells and glial cells.

According to a fifth aspect of the present invention, there is provided a kit for inhibiting the interaction between α-synuclein and FABP3, the kit including the peptide of the first aspect or the antibody of the second aspect of the present invention.

The kit may contain an additional component, such as an additional reagent and/or device for measuring free α-synuclein or aggregation of α-synuclein and FABP, and/or a negative control or a positive control for measuring expression level.

According to a sixth aspect of the present invention, there is provided a preventive or therapeutic agent for prevention or treatment of synucleinopathy, the agent containing the peptide of the first aspect or the antibody of the second aspect as an active ingredient.

According to a seventh aspect of the present invention, there is provided a pharmaceutical composition for prevention or treatment of synucleinopathy, the composition containing the peptide of the first aspect or the antibody of the second aspect.

The synucleinopathy is selected from the group consisting of Parkinson's disease (PD), Lewy body disease (LBD), and multiple system atrophy (MSA). Parkinson's disease includes Parkinson's disease with dementia (PDD), and Lewy body disease includes dementia with Lewy body disease (DLB).

For the preventive or therapeutic agent of the sixth aspect or the pharmaceutical composition of the seventh aspect, a dosage form of various types can be employed; the dosage form may be, for example, any of an injection, an oral formulation, and a nasal formulation, and an injection (such as intravenous, subcutaneous, intradermal, or intraspinal cavity) is preferably employed. These dosage forms can be each manufactured by a formulation method commonly used and known to those skilled in the art.

The target of the administration of the preventive or therapeutic agent of the sixth aspect and/or the pharmaceutical composition of the seventh aspect is a mammal, preferably a mouse, a rat, a hamster, a ferret, a dog, a cat, a monkey, or a human, and more preferably a human.

The preventive or therapeutic agent of the sixth aspect and the pharmaceutical composition of the seventh aspect can contain a pharmaceutical carrier as necessary. For the pharmaceutical carrier, a commonly used organic or inorganic carrier material of various types may be used as a formulation material, and examples include a filler, a binder, a disintegrator, a lubricant, a coating agent, a solvent, a solubilizer, a suspending agent, an isotonic agent, a pH adjuster, a buffering agent, and a micelle agent. In addition, for the preventive or therapeutic agent of the sixth aspect and the pharmaceutical composition of the seventh aspect, a formulation excipient may be used as necessary, such as an antiseptic, an antioxidant, a colorant, a flavoring agent, or a stabilizer.

The amount of the peptide of the first aspect to be contained in each dosage unit form described above varies depending on the symptom of a patient to whom the peptide is to be applied, the dosage form, or the like but is typically from 0.1 ng to 1000 mg and preferably from 10 ng to 10 mg per dosage unit form.

The daily dose of the peptide of the first aspect having the above dosage form varies depending on the symptom, body weight, age, sex, and the like of a patient and cannot be unconditionally determined, but the peptide is typically preferably administered to an adult (body weight 50 kg) at 0.1 ng to 1000 mg daily, preferably at 10 ng to 10 mg once daily or separately approximately twice or three times daily, on a weekly basis, a biweekly basis, a four-week (a monthly) basis, or a bimonthly basis.

The amount of the antibody of the second aspect to be contained in each dosage unit form described above varies depending on the symptom of a patient to whom the antibody is to be applied, the dosage form, or the like but is typically from 0.1 ng to 1000 mg and preferably from 10 ng to 10 mg per dosage unit form.

The daily dose of the antibody of the second aspect having the above dosage form varies depending on the symptom, body weight, age, sex, and the like of a patient and cannot be unconditionally determined, but the antibody is typically preferably administered to an adult (body weight 50 kg) at 0.1 ng to 1000 mg daily, preferably at 10 ng to 10 mg once daily or separately approximately twice or three times daily, on a weekly basis, a biweekly basis, a four-week (a monthly) basis, or a bimonthly basis.

The disclosures of all patent applications and literatures cited in the present specification are incorporated herein by reference in their entirety.

Hereinafter, the present invention will be described more specifically with reference to examples, but the present invention is not limited to these.

### EXAMPLES

### Example 1: Interaction between FABP3 and α-Synuclein Full Length or Partial Peptide (Method)

First, FIGS. 2A and B are descriptions about regions corresponding to a structure of a natural protein α-synuclein. FIG. 2A illustrates region sites of peptides (SynP2-10, SynP73-96, and SynP130-140) used in the present example, and FIG. 2B lists the amino acid sequences of those peptides (SEQ ID NO: 2, SEQ ID NO: 3, and SEQ ID NO: 4).

The interactions of FABP3 with a wild-type α-synuclein (Syn) or peptides (SynP2-10, SynP73-96, and SynP130-140) having a portion of a wild-type α-synuclein were measured using a quartz crystal microbalance (QCM measuring device (AffinixQNµ, Initium) technique. A Ni-NTA method was used to immobilize FABP3 on a quartz substrate. Specifically, a quartz substrate was washed with a 1% SDS and a piranha solution (H₂SO₄:H₂O₂ = 3:1), then 100 µL of a 0.5 mM 3,3'-dithiobis[N-(5-amino-5-carboxypentyl)propionamide-N',N'-diacetic acid]dihydrochloride (C₂-NTA, Dojindo) solution was added, and the substrate was allowed to stand at room temperature for 10 minutes. The substrate was washed with purified water, then 500 µL of a Ni solution (20 mM HEPES-NaOH buffer, pH 7.5, 150 mM NaCl, 50 mM EDTA, 10 mM NiSO₄) was added, and the substrate was allowed to stand at room temperature for 10 minutes. The substrate was washed with purified water, then 100 nM of FABP3 fused with a His-tag at the N-terminus diluted with a 50 mM Tris-HCl pH 7.4 was added to a quartz sensor and treated for one minute to be immobilized. To each His-tagged FABP3 immobilized on a quartz sensor was added 2.5 nM, 5 nM, 10 nM, 15 nM, or 20 nM of each of wild-type α-synuclein and three peptides (SynP2-10, SynP73-96, and SynP130-140) each diluted with a 50 mM Tris-HCl pH 7.4, and the interaction was measured with a QCM device. The measurement was performed in a 500 µL liquid under conditions of 25 °C and 1000 rpm for three minutes. After the measurement, Kd, kon, and koff were each calculated with AQUA 2.0 software (Initium).

### (Results)

FIGS. 3A to D are graphs each showing the interactions of FABP3 with α-synuclein full length (Syn) (A), the SynP2-10 peptide (B), the SynP73-96 peptide (C), and the SynP130-140 peptide (D). As shown in FIG. 3A, α-synuclein full length (Syn) bound to FABP3 in a dose dependent manner. As shown in FIGS. 3B and C, the SynP2-10 peptide of the N-terminal region of α-synuclein and the SynP73-96 peptide of the NAC region of α-synuclein hardly bound to FABP3, and surprisingly, the SynP130-140 peptide of the C-terminal region bound to FABP3 in a dose dependent manner as shown in FIG. 3D. The SynP130-140 peptide is a peptide corresponding to the very C-terminal of the C-terminal region (100-140) of α-synuclein.

### Example 2: Interactions between FABP3 and Various Mutants of SynP130-140 (Method)

For the SynP130-140 peptide, the following mutants were produced: a mutant in which tyrosine at position 133 was changed to phenylalanine (Y133F), a mutant in which tyrosine at position 136 was changed to phenylalanine (Y136F), a mutant in which tyrosine at position 133 was changed to tryptophan (Y133W), a mutant in which tyrosine at position 133 and tyrosine at position 136 were both changed to phenylalanine (Y133F/Y136F), a mutant in which tyrosine at position 133 was changed to phenylalanine and tyrosine at position 136 was changed to tryptophan (Y133F/Y136W), and a mutant (Y136F/Y136W) in which tyrosine at position 133 and tyrosine at 136 were both changed to tryptophan.

FABP3 and various mutants of SynP130-140 were interacted in the same manner as in Example 1. The measurement was performed by adding each peptide at any concentration to the immobilized FABP3.

### (Results)

As shown in FIG. 4A, changing tyrosine at position 133 to phenylalanine resulted in a twofold stronger binding affinity. As shown in FIGS. B and C, changing tyrosine at position 136 to phenylalanine or tryptophan did not strengthen the affinity but maintained about the same affinity for FABP3 as that of the peptide with tyrosine (Y) at position 136. As shown in FIG. 4D, changing tyrosine at two positions to phenylalanine resulted in about the same affinity for FABP3 as that of the mutant (Y133F) with the change at one position, that is, a 2-fold stronger binding affinity of that of the natural sequence. From these results, changing tyrosine at position 133 to phenylalanine appears to be important. As shown in FIG. 4E, changing tyrosine at position 136 to tryptophan in addition to the mutation of tyrosine at position 133 to phenylalanine failed to further strengthen the binding affinity by that much of the additional mutation at position 136. As shown in FIG. 4F, changing tyrosine (Y) at two positions to tryptophan (W) did not change the binding affinity compared with that of the natural sequence. The mutation to tryptophan apparently maintains the affinity but does not necessarily strengthen the affinity.

### Example 3: Suppression of Amyloid Fibril Formation by FABP3 Addition to α-Synuclein and Aggregation Promotion by Peptide Addition (Method)

FABP3, α-synuclein (Syn), and various SynP130-140 mutant peptides were allowed to coexist in a 1:1:1 molar ratio, and amyloid fibril formation of α-synuclein was observed by fluorescence with thioflavin T (Thio-T), or the morphologies of the amyloid fibrils at their final time points were measured with an electron microscope (TEM). An ARVO X4 (Perkin Elmer) was used for shaking and measuring fluorescence values over time in the fibril formation process of Syn. For the measurement, the samples were added to a 96-well plate (8 × 12-well plate; Greiner, Kremsmuenster, Austria) to give 150 µL/well, and the fluorescence (Ex of 440 nm and Em of 486 nm) was measured every 15 minutes while the well plate was continuously shaken at 37°C. For the measurement samples, α-synuclein, FABP3, and the peptide (SynP130-140, SynP130-140 Y133F, Y136F, Y136W, Y133F/Y136F, Y133F/Y136W, or Y133W/Y136W) were each added to give 69 µM in 50 mM Tris-HCl pH 7.4, 150 mM NaCl, and 20 µM Thio-T 500 µL. Samples after 1800 minutes obtained after the measurement were measured with a transmission electron microscope (TEM) (JEOL JEM-1400 Plus). To a collodion membrane (400 mesh; Nisshin EM, Tokyo, Japan), 10 µL of the sample was added and allowed to stand at room temperature for two minutes, then washed with 5 µL of purified water, stained with 5 µL of a 10% EM stain solution for one minute, and then washed with 5 µL of purified water. This was air-dried overnight and then measured.

### (Results)

FIG. 5A is the fluorescence observation over time of the amyloid fibril formation, and FIG. 5B is a series of micrographs showing synuclein aggregation when the same mutant peptides as those used in Example 2 were used. Adding equimolar FABP3 to α-synuclein completely suppressed the aggregation of synuclein (• in the graph). However, when equimolar FABP3 and the peptide of P130-140 (× in the graph) or the peptide with the mutation to F at position 133 (◆ and ▪ in the graph ) are added simultaneously to α-synuclein, the peptide binds to FABP3 before α-synuclein because the peptide has stronger binding to FABP3 (Kd = 0.2 to 0.1 nM) than α-synuclein (full length, Kd = 0.4 nM), and α-synuclein failing to bind to FABP3 forms amyloid fibril aggregates.

### Example 4: Concentration Dependence of SynP130-140 Y133F Peptide on Amyloid Fibril Aggregate Formation

FABP3, α-synuclein (Syn), and the SynP130-140 mutant peptide with tyrosine at position 133 changed to phenylalanine (SynP130-140 Y133F) were allowed to coexist in a 1:1:1 molar ratio, and amyloid fibril formation of α-synuclein was observed by fluorescence with thioflavin T (Thio-T), or the morphologies of the amyloid fibrils at the final time points were measured with an electron microscope (TEM). The thio-T measurement and the measurement with a TEM were performed in the same manner as in Example 3. In the Thio-T measurement, SynP130-140 Y133F was added to be 0.1 equivalents (6.9 µM), 0.5 equivalents (34.5 µM), or 1 equivalent (69 µM) to 69 µM of α-synuclein and 69 µM of FABP3, and the measurement was performed.

### (Results)

FIG. 6A is the fluorescence observation over time of the amyloid fibril formation, and FIG. 6B is a series of micrographs showing synuclein aggregation when the SynP130-140 Y133F peptide was used at various concentrations. Adding equimolar FABP3 to α-synuclein completely suppressed the amyloid fibril aggregation of α-synuclein. When the Y133F peptide (Kd = 0.1 nM) is added simultaneously there to be 0.1 equivalents, 0.5 equivalents, or 1 equivalent, the Y133F peptide preferentially binds to FABP3 in a dose-dependent manner, and α-synuclein failing to bind to FABP3 forms amyloid aggregates. The graph of the closed circles for 0 equivalent is the amyloid fibril aggregate formation when only FABP3 and α-synuclein were present.

### Example 5: Effect of Addition Time of SynP130-140 Peptide on Amyloid Fibril Aggregate Formation

The state of amyloid fibril formation of released α-synuclein when the SynP130-140 peptide was added to the complex of α-synuclein and FABP3 after a delay was observed by fluorescence or with a TEM at the final time.

The thio-T measurement and the measurement with a TEM were performed in the same manner as in Example 3. The Thio-T measurement was performed for samples to each of which SynP130-140 was added at the beginning (0 hour), after 3 hours, 8 hours, or 24 hours.

### (Results)

FIG. 7A is the fluorescence observation over time of the amyloid fibril formation at each addition time, and FIG. 6B is a series of micrographs showing synuclein aggregation when the SynP130-140 Y133F peptide was added at each addition time. Adding equimolar FABP3 to α-synuclein forms a 1:1 Syn-FABP3 complex. Adding equimolar C-terminal peptide (P130-140) to the complex after a delay at any of times indicated by arrows releases Syn from FABP3 because P130-140 has stronger affinity for FABP3, and SynP130-140 binds to FABP3. Syn released from FABP3 was observed to form amyloid fibril aggregates.

### Example 6: Uptake of α-Synuclein and Uptake Antagonistic Effect by C-Terminal Peptide in Dopaminergic Nerve Cells

### (Method)

Cultured dopaminergic nerves were prepared according to the method (Kawahata et al. 2019) already described. Specifically, the uterus was aseptically removed from a C57BL6N mouse on day 15.5 of pregnancy by abdominal midline incision under deep isoflurane anesthesia, and the fetal mesencephalon was removed in ice-cold Hank's balanced salt solution (HBSS) under a stereomicroscope. The tissue was then dispersed by treatment with a nerve cell suspension (Sumitomo Bakelite) at 37°C for 30 minutes, the dispersion was centrifuged at 1000 rpm for 5 minutes, and then the supernatant was discarded. The cell pellet was dispersed, then a removal solution (Sumitomo Bakelite) was added, and the mixture was centrifuged at 900 rpm for 5 minutes. The supernatant was then removed, the pellet was suspended in Eagle's minimal essential medium (EMEM) and seeded on a poly-L-lysine-coated chamber plate (Iwaki). On day 10 of culture, for visualization analysis of α-synuclein, 1 µM ATTO-550-labeled α-synuclein monomer was added to the medium, and the treatment was performed for 48 hours in the coexistence of a peptide of a synthesized α-synuclein C-terminal 20 residues DNEAYEMPSEEGYQDYEPEA (1 µM) (SEQ ID NO: 6) or 10 residues EGYQDYEPEA (1 µM) (SEQ ID NO: 6), or in the non-coexistence. The antagonistic effect by the peptide on the uptake of α-synuclein into dopaminergic nerves was evaluated by quantitative analysis of the intracellular fluorescence intensity of ATTO-550-labeled α-synuclein (NIH Image J software). Values are mean ± SEM (n > 20), and one-way ANOVA with Tukey test was performed for all the groups. The conceptual diagram of the synthesized α-synuclein is illustrated in C.

### (Results)

The cultured dopaminergic nerves took up the ATTO-550-labeled α-synuclein into the cells (FIG. 8A, control, red), but the simultaneous treatment with the synuclein C-terminal peptide 20 residues or 10 residues inhibited the intracellular uptake of the fluorescently labeled α-synuclein (FIG. 8A,20aa, 10aa, FIG. 8B). **** indicates p < 0.0001 in comparison with the control group (control) (FIG. 8B). No significant difference was observed between the synuclein C-terminal peptide 20 residues and 10 residues in the inhibitory effect on the synuclein uptake. These peptides belong to the synuclein C-terminal region (FIG. 8C).

### Example 7: Characteristics of Intracellular Uptake of Wild-Type α-Synuclein and C-Terminal Deletion Mutant in Dopaminergic Nerve Cells

### (Method)

The cultured dopaminergic nerves were prepared in the same manner as described above (Example 6). On day 10 of culture, for visualization analysis of α-synuclein, 1 µM ATTO-550-labeled α-synuclein monomer or an ATTO-550-labeled α-synuclein monomer in which the synuclein C-terminal 130-140 residues were deleted on the contrary to Example 6 was added to the medium, and their uptake into nerve cells was examined. The uptake amount of the wild-type α-synuclein or the C-terminal 130-140 residue deletion α-synuclein into dopaminergic nerves was evaluated by quantitative analysis of the intracellular fluorescence intensity of ATTO-550-labeled α-synuclein (NIH Image J software). Values are mean ± SEM (n > 20), and two groups were compared by Student's T-test. The conceptual diagram of the synthesized C-terminal 130-140 residue deletion α-synuclein is illustrated in FIG. 9C.

### (Results)

The cultured dopaminergic nerves took up the ATTO-550-labeled α-synuclein into the cells (αS, WT), but the C-terminal 130-140 residue deletion α-synuclein (αS, Δ130-140) was not taken up into the nerve cells (FIG. 9A, red, B). **** indicates p < 0.0001 in comparison with the control group (control) (FIG. 9B).

### Example 8: Binding Experiment of FABP and α-Synuclein Peptides

### (Method)

For the affinity of the interaction between FABP3 and various peptides or α-synuclein, quantitative values were determined using a QCM measuring device. The detailed experimental method is as described in the method of Example 1.

### (Results)

The binding results of FABP3 and various peptides are as shown in FIG. 10. The results showed that the C-terminal 130-140 residues of α-synuclein is involved in the binding of FABP3 and α-synuclein. In addition, the results of the mutant of FABP3 and the mutant of the α-synuclein C-terminal peptide showed that the phenyl group of the 16th phenylalanine in the amino acid sequence of the FABP3 protein interact with the phenyl group, tyrosine, or tryptophan in position 133 and/or the phenyl group, tyrosine, or tryptophan in position 136 of α-synuclein.

[Sequence Listing]

## Claims

1. A peptide represented by (i) or (ii) below:
(i) a peptide comprising an amino acid sequence represented by EEG(X1)QD(X2)EPEA, wherein X1 and X2 are identical or different and are Y, F, or W; or
(ii) a peptide comprising an amino acid sequence where one or several amino acids are deleted, substituted, or added in amino acid positions excluding X1 and X2 of the amino acid sequence of (i) above and having an activity of binding to FABP3 protein.

2. The peptide according to claim 1, to bind to FABP3 through an interaction between phenyl groups of the amino acids of X1 and X2 and a phenyl group of 16th phenylalanine in an amino acid sequence of native FABP3 protein.

3. An antibody against the peptide according to claim 1 or 2.

4. A preventive or therapeutic agent for prevention or treatment of synucleinopathy, the agent comprising the peptide according to claim 1 or 2 or the antibody according to claim 3 as an active ingredient.

5. The preventive or therapeutic agent according to claim 4, wherein the synucleinopathy is selected from the group consisting of Parkinson's disease, Lewy body disease, and multiple system atrophy.

6. A pharmaceutical composition for prevention or treatment of synucleinopathy, the composition comprising the peptide according to claim 1 or 2 or the antibody according to claim 3.
